(19) Europäisches Patentamt
European Patent Office
Office européen des brevets

(11) EP 4 423 499 B1

(12) EUROPEAN PATENT SPECIFICATION

(45) Date of publication and mention of the grant of the patent:
**09.09.2026 Bulletin 2026/37**

(21) Application number: **22817340.7**

(22) Date of filing: **31.10.2022**

(51) International Patent Classification (IPC):
***G01N 33/483*** *(2006.01)* ***B01L 1/02*** *(2006.01)*
***C12M 1/34*** *(2006.01)*

(52) Cooperative Patent Classification (CPC):
**G01N 33/4833; A61K 35/42; B01L 1/025; B01L 3/508; B33Y 80/00;** B01L 2300/047; B01L 2300/048; G01N 33/5014

(86) International application number:
**PCT/IB2022/060464**

(87) International publication number:
**WO 2023/073658 (04.05.2023 Gazette 2023/18)**

# (54) PORTABLE DEVICE TO STUDY THE EXPOSURE OF CELLS TO DRY POWDERS

TRAGBARE VORRICHTUNG ZUR UNTERSUCHUNG DER EXPOSITION VON ZELLEN GEGENÜBER TROCKENPULVERN

DISPOSITIF PORTATIF POUR L'ÉTUDE DE L'EXPOSITION DE CELLULES À DES POUDRES SÈCHES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **29.10.2021 PT 2021117541**

(43) Date of publication of application:
**04.09.2024 Bulletin 2024/36**

(73) Proprietors:
- **Universidade Do Algarve**
  **8005-139 Faro (PT)**
- **Centro de Ciências Do Mar Do Algarve (CCMAR)**
  **8005-139 Faro (PT)**
- **Instituto Superior Técnico**
  **1049-001 Lisboa (PT)**

(72) Inventors:
- **MOUTINHO GRENHA, Ana Margarida**
  **8005-139 FARO (PT)**
- **RODRIGUES PONTES, Jorge Filipe**
  **8005-139 FARO (PT)**
- **DA SILVA COELHO BORGES DOS SANTOS, Rui Miguel**
  **8005-139 FARO (PT)**
- **ENCARNAÇÃO DA CONCEIÇÃO, Eusébio Zeferino**
  **8005-139 FARO (PT)**
- **PIRES DIOGO, Hermínio Albino**
  **1049-001 LISBOA (PT)**

(74) Representative: **Patentree**
**Edificio Net**
**Rua de Salazares, 842**
**4149-002 Porto (PT)**

(56) References cited:
**WO-A1-2021/104787 US-A1- 2018 171 280**

- **THORNE DAVID ET AL: "A review of in vitro cigarette smoke exposure systems", EXPERIMENTAL AND TOXICOLOGIC PATHOLOGY., vol. 65, no. 7-8, 1 November 2013 (2013-11-01), DE, pages 1183 - 1193, XP093016972, ISSN: 0940-2993, DOI: 10.1016/j.etp.2013.06.001**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KEYSER BRIAN M. ET AL: "Investigation of multiple whole smoke dosimetry techniques using a VITROCELL VC10 smoke exposure system", TOXICOLOGY REPORTS, vol. 6, 1 January 2019 (2019-01-01), pages 1281 - 1288, XP093016977, ISSN: 2214-7500, DOI: 10.1016/j.toxrep.2019.10.011
- CHANDRALA LAKSHMANA D. ET AL: "A Device for measuring the in-situ response of Human Bronchial Epithelial Cells to airborne environmental agents", SCIENTIFIC REPORTS, vol. 9, no. 1, 13 May 2019 (2019-05-13), pages 1 - 12, XP093016968, Retrieved from the Internet <URL:https://www.ncbi.nlm.nih.gov/pmc/articles/PMC6513995/pdf/41598_2019_Article_43784.pdf> DOI: 10.1038/s41598-019-43784-5

## Description

### Technical domain

The present disclosure relates to a portable device to study the exposure of cells to dry powders, and the use thereof.

### Background

The paradigm of drug administration has been changing for the past years, as alternative routes, new technologies, and strategies have been widely studied to improve therapeutic outcomes [1,2]. Lung drug delivery has been considered a relevant alternative for the treatment of local diseases and for the systemic delivery of drugs. Nevertheless, the approach requires complying with aerodynamic requisites to reach the appropriate area of the lung [3]. Additionally, different regions of the respiratory tract present different physiological environments: the bronchi have a mucus layer, while the alveoli are completely devoid of liquid, being covered only by a thin layer of lung lining fluid preventing desiccation [4]. This contrasts with more conventional routes, such as the oral, characterised by abundant volume of liquid surrounding the mucosal tissue, making the evaluation of behaviours of inhalable drug formulations challenging. *In vitro* tests comprise the first line of assessment to ascertain the potential of drug formulations and are expected to provide robust and indicative information on the formulation performance. Dry powders are currently of the most popular inhalable formulations, but their evaluation in cell-based assays is complex, given the difficulties in mimicking the lung environment. Conventional cell-based experiments involve considerable volume of liquid, in which cells are cultured with liquid on the apical side. Dry powders are often described to be simply dispersed in the liquid for cell exposure, which does not resemble *in vivo* conditions in the specific case of the lung route. Delivering dry powders as such is, in fact, a difficult task.

The market provides some adequate solutions to tackle the *in vitro* testing of dry powders, all involving quite complex and expensive equipment not easily accessible to most of the inhalation community.

Document WO2011003022 discloses a laboratory animal pharmaceutical testing device comprising a substantially closed animal holding cell, having a dry powder generator communicating through a wall of the cell, and one or more filtered inlets for permitting exchange of fresh air into the cell. In this technical solution, a pharmaceutical nebulizer communicates through a side wall of an animal holding cell. The nebulizer preferably comprises a dry powder nebulizer and communicates via conduit through an opening in side wall that forms an essentially airtight seal. In this embodiment, the opening preferably is located in the upper part of animal holding cell so that a cloud of powdered pharmaceutical formulation will be injected into the upper part of cell and the bulk of the powder will be inhaled by the animal or animals in the cell before the powder settles to the bottom of the cell. In that way, unused medication is returned rather than wasted.

Document US2010083737A1 discloses a device for measuring superfine particle masses including a quartz oscillator and an exposure system having at least two measuring chambers. Each of the at least two measuring chambers has a same geometry, a deposition surface for particles, and an aerosol feed directed at the respective disposition surface configured to feed an aerosol onto the respective deposition surface. At least one of the respective deposition surfaces is disposed on the quartz oscillator.

Commercially available aerosol exposure systems are discussed in the journal article: "A review of in vitro cigarette smoke exposure systems", Experimental and Toxicologic Pathology, 2013, volume 65, pages 1183-1193, authors David Thorne and Jason Adamson; focusing on aerosol generation, dilution, dosimetry, and system limitations.

Therefore, none of the documents cited from the prior art present a portable and easy to use device.

These facts are described to illustrate the technical problem solved by the embodiments of the present document.

### General Description

The present disclosure relates to a portable device according to independent claim 1 to study, i.e. to determine or measure, the exposure of cells to dry powders.

The present disclosure relates to a portable device to study the exposure of cells to dry powders comprising a hollow main body, a hollow base, a headspace and at least an inlet for gas flow, wherein the main body has bell format; wherein the bottom of the main body is sufficiently large to fit a cell support; wherein the headspace has a form of inverted cone shape; wherein the headspace is bound to the main body; wherein the bell format of main body comprises an angle between the external wall of the upper part of the bell format to the vertical defined by the tangent to the outside wall of the bottom of the main body equal or below to 20° for allow the turbulent regime of the gas injected, allowing the drag and deposition of a dry powder over the cells in the cell support.

The technology now disclosed aims to overcome the market limitation of lack of a simple device that provides an

adequate solution to tackle the *in vitro* testing of dry powders. That limitation impelled us to design, conceive and develop an easy-to-use and economically accessible device that allows the aerosolization of dry powders, providing homogenous dispersion over a cell support. This cell support can serve various purposes, including the culturing of cells at a later stage. The device targets initial stages of formulation development frequently impaired by the inability to afford high-cost equipment. Its use is expected to allow refining and selecting drug formulations for more advanced stages. The now disclosed device is technically simpler and has a much lower cost in comparison with other alternatives in the market and even may have broader applications. If cells are cultured in the cell support, apart from drug delivery related applications, others related to the evaluation of the impact of pollution or electronic cigarettes in the human respiratory system may apply.

The mentioned cell support, under the present application, can be a plate, a Petri dish or a quartz crystal microbalance, to enable the study of deposition profiles.

One aspect of the present disclosure is a portable device to study, i.e. to determine or measure, the exposure of cells to dry powders comprising a hollow main body, a hollow base, a headspace and at least an inlet for gas flow, wherein the main body has bell format; wherein the bottom of the main body is sufficiently large to fit a cell support; wherein the headspace has a form of invert cone shape; wherein the headspace is bound to the main body; wherein the bell format of main body comprises an angle between the external wall of the upper part of the bell format to the vertical defined by the tangent to the outside wall of the bottom of the main body equal or below to 20° for allow the turbulent regime of the gas injected, allowing the drag and deposition of a dry powder over the cells in the cell support.

The device of the present disclosure surprisingly allow the correct distribution of the tested dry powder over the cells, without introduction of fluids and preventing cell damage.

In an embodiment, the main body of the portable device comprises a bell format with an angle to the vertical defined by the tangent to the outside wall of bottom of the main body ranging from 15° to 19°, more preferably from 16° to 18°, even more preferably from 17° and 18°.

In accordance with the invention, the headspace of the portable device comprises a powder support for loading dry powders, preferably wherein said powder support is perforated to retain the powders and allow the powders dispersion with the air flow.

In an embodiment, the headspace of the portable device comprises a plateau for loading dry powders; preferably, the headspace is hollow, and at the middle, the plateau is placed containing the dry powders, allowing their dispersion with the air flow.

In an embodiment, the inlet for gas flow of the portable device comprises a channel for gas injection.

In an embodiment, the portable device comprises a seal in the top of the headspace for prevent the escape of the dry powders.

In an embodiment, the powder support of the portable device is at the same height of the inlet for gas flow.

In an embodiment, the pressure of the dry fluid used in the portable device is ranging from 200 MPa to 415 MPa, preferably ranging from 210 MPa to 310 MPa, more preferably ranging from 240 MPa to 290 MPa.

In an embodiment, the main body of the portable device comprises a height up to 20 cm, preferably ranging from 5 to 20 cm and more preferably from 10 to 20 cm.

In an embodiment, the bottom of the main body used in the portable device comprises up to 4.15 cm, preferably ranging from 4.10 to 3.85 cm, more preferably ranging from 3.90 to 4.00 cm of external diameter and ranging from 3.65 to 3.80 cm, preferably ranging from 3.70 to 3.75 cm of internal diameter.

In an embodiment, the inlet for gas flow of the portable device is ranging from 1.5 and 4.0 cm, preferably ranging from 2.0 to 3.5 cm, more preferably ranging from 2.5 and 3.25 cm length and a width ranging from 0.3 and 0.8 cm, preferably from 0.4 and 0.7 cm, even more preferably from 0.5 and 0.6 cm.

In an embodiment, the dry fluid used in the portable device is air.

In an embodiment, the cell support used in the portable device is a plate or a petri dish or a quartz crystal microbalance.

In an embodiment, the portable device is made a polymeric material and/or composite material selected from a list consisting of: acrylonitrile butadiene styrene, polylactic acid, washable resin, epoxy or their combinations.

In an embodiment, the portable device is obtained by additive manufacturing.

In an embodiment, the portable device is obtained by fusion deposition modelling, stereolithography, 3D printing or moulding.

In an embodiment, the portable device further comprises a semi-transparent, flexible film composed of a blend of waxes and polyolefins in the connection between the powder support and the main body.

In an embodiment, the main body of the portable device is anchored at the bottom over a cell support material by gravity.

In an embodiment, the powder support and /or the seal used in the portable device are in a disposable material.

Another aspect of the present disclosure is a method for in vitro testing of dry powders comprising the use of the portable device hereby described.

## Brief Description of the Figures

The following figures provide preferred embodiments for illustrating the disclosure and should not be seen as limiting the scope of invention.

**Figure 1:** Schematic representation of the concept supporting the design and development of a portable device to study the exposure of cells to dry powders to be used in vitro cell-based assays.

**Figure 2:** Schematic representation of the main body (A) and the headspace (B).

**Figure 3:** Schematic representation of a 3D-printed prototype of an embodiment of the device and its use in the aerosolization assays: (A) the device assembled and prepared for an assay and (B) the different parts of the device, after an assay.

**Figure 4:** Graphic representation of aerosolization yields obtained with the device prototype, for microparticles (MP) composed of xanthan gum (XG), xanthan gum/sodium chloride (XG-Na) and dextran sulfate (DS). Results are presented as mean $\pm$ standard deviation, n = 10.

**Figure 5:** Graphic representation of viability of A549 cells, representative of alveolar epithelium, 3h or 24h after exposure to air insufflation (D1-Air and D2-Air) or locust bean gum microparticles (D1- Air + MP LBG S 130 and D2 - Air + MP LBG S 130). Devices D1 and D2 were used to perform the insufflation of only air and of the dry powders (microparticles).

**Figure 6:** Photographs of powder dispersion with the device developed according to this application made from 3D printing process using polylactic acid (PLA) (A) and stereolithography using resin (B).

**Figure 7:** Schematic representation of a 3D-printed prototype of an embodiment of the main body of the device with the indication of the angle $\alpha$ between the upper part of the bell format and the vertical defined by the tangent to the outside wall of the bottom of the main body.

## Detailed Description

The present disclosure refers to a portable device to study; i.e. to determine or measure the exposure of cells to dry powders.

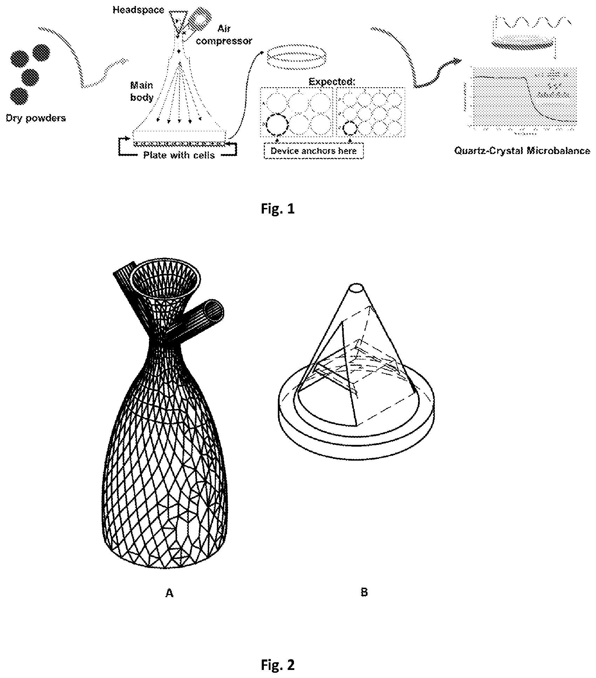

In an embodiment, the portable device to study the exposure of cells to dry powders, for example for dry powder aerosolization onto a cell support, which is suitable for cell culture, was developed and the prototype printed in 3D. Polysaccharide-based microparticles prepared by spray-drying and devoid of any drug (unloaded microparticles) were used to test the device. The yield of the aerosolization process was calculated and air flow parameters are currently being optimised using a quartz crystal microbalance (QCM) [5], which will enable subsequent assessment of the deposition profile of selected dry powders. An embodiment of the portable device of the present disclosure is represented in Figure 1. From left to right, dry powders are weighed and loaded into a powder support that is subsequently assembled with the main body of the portable device. A dry fluid, such as compressed air, is then loaded into the main body of the device through an inlet, dragging the powder from the powder support, and dispersing it over cells cultured in a cell support, such as a plate, a petri dish or a quartz crystal microbalance. The dry powder deposition profile is further analysed using a quartz crystal microbalance, through differences in the crystal vibration frequency. The zone that is designed to have the powder in the powder support must be aligned/at the same height with the entry of air, to maximise the amount of powder that is dragged. The main body is anchored at the bottom over a cell support material, for example by simple placement by gravity.

In an embodiment, the powder support for loading dry powers within the headspace of the portable device can be perforated to retain the powders and allow the powders dispersion with the air flow.

In an embodiment, a functional prototype of a portable device to study the exposure of cells to dry powders able to disperse dry powders onto a plate was designed and 3D printed successfully, using a biodegradable/biocompatible polymer. The usage of the device required using a dry fluid provider, such as an air compressor, and protocol optimisation established the need to perform 30 quick air blows to maximise powder dispersion. Nevertheless, the tests show that in order to get a satisfactory powder dispersion at least 10 quick air blows are sufficient. Moreover, testing different air pressures resulted in the indication of air pressures between 200 MPa and 415 MPa, preferably between 210 MPa and 310 MPa, more preferably between 240 MPa and 290 MPa, which provide higher aerosolization yield. Yields up to 51% were obtained, which were observed to depend on particle tenuity, with lower tenuity facilitating the dispersion process. To further optimise the process, the deposition profile is being studied using quartz crystal microbalance, a highly accurate

balance enabling more precise and real-time determinations. Moreover, to push forward the device, it is possible to adapt the main body of the device to plates, Petri dishes and other types of cell supports, including 6-well and 12-well plates, which is being performed.

Design and printing process

In an embodiment, the portable device of the present disclosure can be produced using a commercial software and exported to a commercial 3D printer, equipped with a standard 0.4 mm diameter double extruder nozzle but used in single mode. The apparatus was comprised of an extrusion system and a heated printing bed, which works synergistically with the extruded polymer, in filament form of 2.85 mm, at high temperatures. Printing was performed at 225°C using biodegradable poly-lactic acid. The nozzle height was set at 1.0 mm, with layers of 0.2 mm being applied. The extrusion speed was set at 17 mm/s.

In an embodiment, the portable device of the present disclosure can be produced comprising two parts, one comprising a small headspace, where the dry powder is to be weighed and loaded, and another as the main body, conceived to allow the powder to be dragged and deposited over cell layers.

In an embodiment, the material of the portable device of the present disclosure can be a polymeric or a composite material, such as acrylonitrile butadiene styrene, polylactic acid, washable resin, epoxy or their combinations. In terms of manufacturing processes, one can use 3D printing, fusion deposition modelling, stereolithography or moulding.

Aerosolization of powders

In an embodiment, polysaccharide-based (unloaded) microparticles for lung applications, irregularly shaped spheres, either hollow or solid, composed, for example, of xanthan gum, konjac glucomannan, carrageenan, chondroitin sulfate, locust bean gum and dextran sulfate, among others that are suitable to be used, and produced by spray-drying were selected as model dry powders to test the device [6,7,8,9]. Before the assay, all parts of the device and a plate suitable for cell culture were weighed. Afterwards, 5 - 10 mg of dry powder were weighed in the headspace, the device assembled, and the powder aerosolized after blowing air into the device. The outlet pressure of the air compressor varied between 200 MPa and 415 MPa. After conclusion of the assay, the parts of the device and the plate were weighed, and images of the plate recorded after powder deposition. The yield of aerosolization was calculated as follows:

Yield of aerosolization (%) = (Amount of powder on the cell support material / Total amount of powder weighed) * 100.

Evaluation of the profile of dry powder deposition by quartz crystal microbalance

Maxtek quartz crystals (5MHz, Inficon), coated with optically flat polished gold electrodes on both sides, were cleaned before use with absolute ethanol, ultrapure water and a piranha solution comprised of 3:1 mixture of sulphuric acid and 30% hydrogen peroxide for 15 min. Cleaned crystals were mounted on a kynar crystal holder (Maxtek CHT-100). The 3D-printed device was placed over it, and the aerosolization assay performed as described above. Changes in the vibration frequency of the quartz crystal were registered with a universal frequency counter (Agilent 53131A) through a phase-locked oscillator (Maxtek PLO-10i). The frequency counter is connected by GPIB to a computer running a data acquisition and control program developed in VEE V9.0 (Agilent) for the real time acquisition of the QCM frequency.

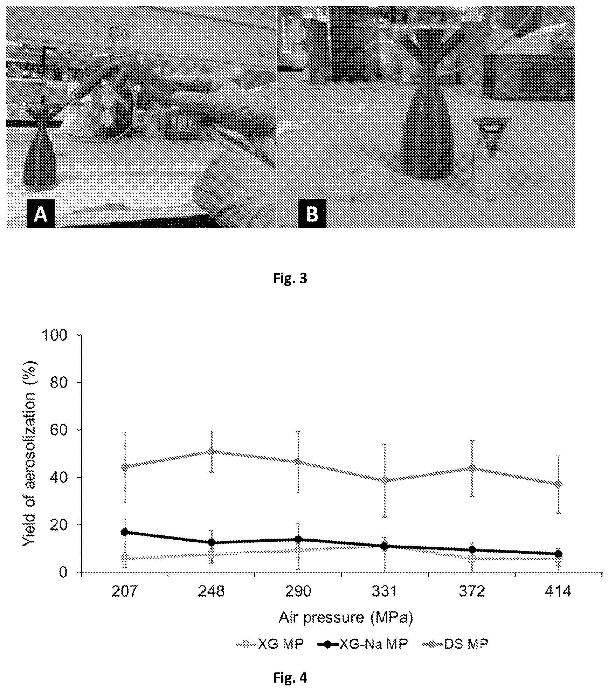

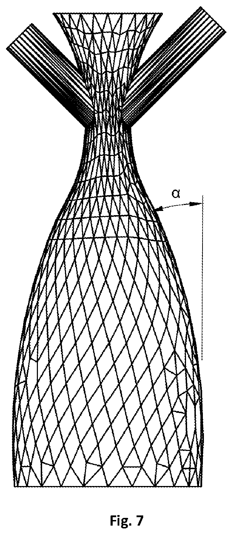

In an embodiment, following the portable device proposed in Figure 1, the device shown in Figure 2 was designed and prepared using the software AutoCAD. The final 3D-printed prototype is shown in Figure 3B. The device shall present a height up to 20 cm, preferably ranging from 5 to 20 cm and more preferably from 10 to 20 cm, and the bottom of the main body has up to 4.15 cm, preferably ranging from 4.10 to 3.85 cm, more preferably ranging from 3.90 to 4.00 cm of external diameter and ranging from 3.65 to 3.80 cm, preferably ranging from 3.70 to 3.75 cm of internal diameter, to fit into the plate having a 3.6 cm of diameter. The dimensions of the bottom are adjustable to allow adapting to other supports. The dry fluid forced inlet, which allows the entry of air into the main body of the device, is ranging from 1.5 and 4.0 cm, preferably ranging from 2.0 to 3.5 cm, more preferably ranging from 2.5 and 3.25 cm length and a width ranging from 0.3 and 0.8 cm, preferably from 0.4 and 0.7 cm, even more preferably from 0.5 and 0.6 cm. The main body of the device has a bell format with an angle between the external wall of the upper part of the bell format to the vertical defined by the tangent to the outside wall of the bottom of the main body equal or below 20°, preferably ranging from 15° to 19°, more preferably from 16° to 18°, even more preferably from 17° to 18°. This angle, illustrated in Figure 7, ensure that the desired air flow regime is obtained to drag the powder from the headspace, and dispersing it over cells cultured in a cell support material.

In an embodiment, one of the important steps of the aerosolization process is the strength of the applied air flow, which should be enough to disperse all the powder but not excessive to induce destabilisation of the cell layer. In the first stages of development, various sources were tested for blowing air: lens cleaner, syringe, bicycle pump and a gardening sprayer. The latter performed better, but it was concluded that the strength of the air flow was not enough to adequately

disperse dry powders onto the plate. Therefore, an air compressor was tested to enhance the amount of dispersed powder. The aerosolization protocol was optimised, establishing a quick blow of air 30 times with an air blow gun (Figure 3A) as the most effective way to ensure that all the powder is dragged to the plate.

In an embodiment, the following step involved determining the ideal operating air pressure to be set in the air compressor, aiming at two objectives: 1) maximise the amount of dispersed powder and 2) generate enough flow intensity without forcing powder dispersion to outside of the testing device. Various intensities of air pressure were tested to aerosolize different dry powders, all developed for inhalation purposes, and results are depicted in Figure 4.

The obtained results indicate that pressures of 248 MPa and 290 MPa are those maximising the yield of aerosolization, that is, the amount of powder leaving the powder support onto the cell support material. Observations of tests performed at different air pressure indicated that dry powders comprised of particles with higher tenuity have facilitated dispersion, compared with finer particles.

Optimizing the air flow that goes into the device is critical for the quality of the aerosolization process and powder deposition. Quartz crystal microbalance can be very helpful in this regard as, unlike a conventional balance, it allows following the deposition in real-time with great accuracy. In this way, the mass deposition *per* air blow can be followed to further optimize the procedure. To this end, the developed device was assembled on top of a quartz crystal microbalance, which is replacing the plate, to determine the deposition profile of aerosolized dry powders at different air flow pressures.

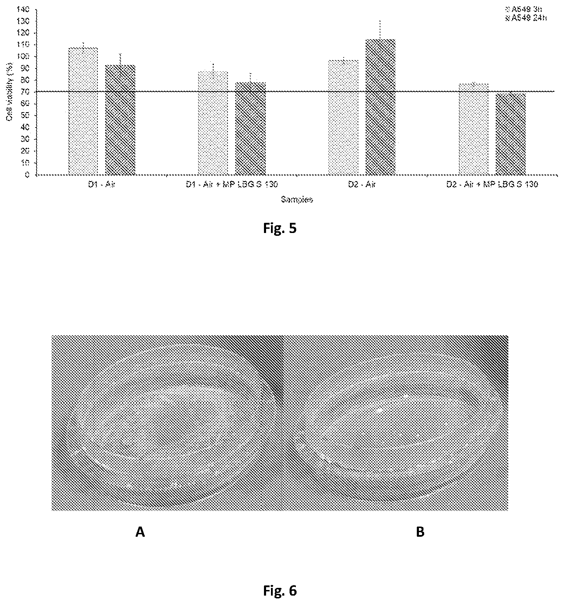

The exposure of cells to dry powders resulted in cell viabilities above 70%. In accordance with the ISO guideline 10993-5 (2009 - Biological evaluation of medical devices - Part 5: Tests for *in vitro* cytotoxicity), it means that this device and the insufflation process can be used safely and without significant loss of cell viability, as illustrated by the results of Figure 5.

As for the powder deposition profiles, a photograph of an assay employing a dry powder and the two tested devices show that the powder is much more homogenously dispersed, when compared to the prior art where the powder is deposited on the centre of the petri dish. Figure 6 highlight, also, some spots of dry powder, but that behaviour is observed with all the powders that were tested. Moreover, the used type of powder influences the dispersion pattern. For example, if a finer particle dry powder is used, the dispersion will resemble the one showed in the first photo (Figure 6A), with powder on the bottom and a bit on the laterals of the petri dish.

In some embodiments and in order to increase the isolation from the headspace to the main body, a semi-transparent, flexible film composed of a blend of waxes and polyolefins can be used.

A lateral cylinder similar to the inlet can be included in the main body for support and/or balance purposes.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following claims further set out particular embodiments of the disclosure.

This work was developed in the framework of the funding provided from the Portuguese Foundation for Science and Technology (UIDB/04326/2020), and the PhD scholarship to Jorge F. Pontes (PD/BD/137064/2018).

## References

[1] He Y, Liang Y, Han R, Lu W-L, Mak J C W, Zheng Y. Rational particle design to overcome pulmonary barriers for obstructive lung diseases therapy. J Control Release 2019; 314: pp48-61;

[2] Ziaee A, Albadarin A B, Padrela L, Femmer T, O'Reilly E, Walker G. Spray drying of pharmaceuticals and biopharmaceuticals: Critical parameters and experimental process optimization approaches. Eur J Pharm Sci 2019; 127: pp300-18;

[3] Molavi F, Barzegar-Jalali M, Hamishehkar H. Polyester based polymeric nano and microparticles for pharmaceutical purposes: A review on formulation approaches. J Control Release 2020; 320: pp265-282;

[4] Fröhlich E, Mercuri A, Wu S, Salar-Behzadi S. Measurements of deposition, lung surface area and lung fluid for simulation of inhaled compounds. Front Pharmacol 2016; 7: 181;

[5] Ding Y, Weindl P, Lenz A G, Mayer P, Krebs T, Schmid O. Quartz crystal microbalances (QCM) are suitable for real-time dosimetry in nanotoxicological studies using VITROCELL®Cloud cell exposure systems. Part Fibre Toxicol 2020; 17: 44;

[6] Konjac Glucomannan: Guerreiro, F.; Pontes, J.F.; Rosa da Costa, A.M.; Grenha, A. Spray-drying of konjac glucomannan to produce microparticles for an application as antitubercular drug carriers. Powder Technology 2019, 342, 246-252, doi:10.1016/j.powtec.2018.09.068;

[7] Carrageenan: Rodrigues, S.; Cunha, L.; Rico, J.; Rosa da Costa, A.M.; Almeida, A.J.; Faleiro, M.L.; Buttini, F.; Grenha, A. Carrageenan from red algae: An application in the development of inhalable tuberculosis therapy targeting the macrophages. Drug Delivery and Translational Research 2020, 10, 1675-1687, doi:10.1007/s13346-020-00799-0;

[8] Chondroitin sulfate: Rodrigues, S.; Rosa da Costa, A.M.; Fernández-Flórez, N.; Torres, M.D.; Faleiro, M.L.; Buttini, F.; Grenha, A. Inhalable spray-dried chondroitin sulphate microparticles: Effect of different solvents on particle properties and drug activity. Polymers 2020, 12, doi:10.3390/polym12020425;

[9] LBG: Alves, A.D.; Cavaco, J.S.; Guerreiro, F.; Lourenco, J.P.; Rosa da Costa, A.M.; Grenha, A. Inhalable antitubercular therapy mediated by locust bean gum microparticles. Molecules 2016, 21, 702, doi:10.3390/molecules21060702.

## Claims

1. Portable device adapted to determine the exposure of cells to dry powders comprising a hollow main body, a hollow base, a headspace and at least a gas flow inlet, wherein the main body has a bell form,

    wherein the bottom of the main body is adapted to fit a cell support;
    wherein the headspace has the form of an inverted cone shape;
    wherein the headspace is bound to the main body;
    wherein the bell form of the main body comprises an angle between the external wall of the upper part of the bell from to the vertical defined by the tangent to the outside wall of the bottom of the main body equal to or below 20° and which is adapted to facilitate injection of turbulent gas, and which is configured to deposit the dry powder over cells cultured in the cell support; wherein the headspace comprises a powder support for loading the dry powders; preferably wherein said powder support is perforated to retain the powders and facilitate powder dispersion with the air flow.

2. Portable device according to the previous claim, wherein the main body comprises a bell form with an angle to the vertical defined by the tangent to the outside wall of bottom of the main body ranging from 15° to 19°, more preferably from 16° to 18°, even more preferably from 17° and 18°.

3. Portable device according to any of the previous claims, wherein the inlet for gas flow comprises a channel for gas injection.

4. Portable device according to any of the previous claims, comprising a seal in the top of the headspace to prevent the escape of the dry powders.

5. Portable device according to any of the previous claims, wherein the powder support is positioned at the same height as the gas flow inlet, and furthermore wherein the powder support and /or the seal are made from a disposable material.

6. Portable device according to any of the previous claims, wherein the pressure of the dry fluid ranges from 200 MPa to 415 MPa, preferably ranging from 210 MPa to 310 MPa, more preferably ranging from 240 MPa to 290 MPa.

7. Portable device according to any of the previous claims, wherein the main body comprises a height up to 20 cm, preferably ranging from 5 to 20 cm and more preferably from 10 to 20 cm, in particular wherein the main body is anchored at the bottom over a cell support material by gravity.

8. Portable device according to any of the previous claims, wherein the bottom of the main body comprises up to 4.15 cm, preferably ranging from 4.10 to 3.85 cm, more preferably ranging from 3.90 to 4.00 cm of external diameter and ranging from 3.65 to 3.80 cm, preferably ranging from 3.70 to 3.75 cm of internal diameter.

9. Portable device according to any of the previous claims, wherein the inlet for gas flow ranges from 1.5 to 4.0 cm, preferably ranging from 2.0 to 3.5 cm, more preferably ranging from 2.5 to 3.25 cm length and a width ranging from 0.3 to 0.8 cm, preferably from 0.4 to 0.7 cm, even more preferably from 0.5 to 0.6 cm.

10. Portable device according to any of the previous claims, wherein the dry fluid is air.

11. Portable device according to any of the previous claims, wherein the cell support is a plate or a petri dish or a quartz crystal microbalance.

12. Portable device according to any of the previous claims, wherein the portable device is made of a polymeric material and/or composite material selected from a list consisting of: acrylonitrile butadiene styrene, polylactic acid, washable resin, epoxy or their combinations.

13. Portable device according to any of the previous claims wherein the portable device is obtained by additive manufacturing, in particular obtained by fusion deposition modelling, stereolithography, 3D printing or moulding.

14. Portable device according to any of the previous claims, further comprising a semi-transparent, flexible film composed of a blend of waxes and polyolefins in the connection between the powder support and the main body.

15. Method for *in vitro* testing of dry powders comprising the use of the portable device according to any of the previous claims.

**Patentansprüche**

1. Tragbare Vorrichtung ausgelegt zur Bestimmung der Exposition von Zellen gegenüber Trockenpulvern, umfassend einen hohlen Hauptkörper, einen hohlen Sockel, einen Gasraum sowie mindestens einen Einlass für einen Gasstrom,

   wobei der Hauptkörper glockenförmig ist,
   wobei der Boden des Hauptkörpers so ausgelegt ist, dass er auf einen Zellträger passt;
   wobei der Gasraum die Form eines umgekehrten Kegels hat;
   wobei der Gasraum mit dem Hauptkörper verbunden ist;
   wobei die Glockenform des Hauptkörpers einen Winkel zwischen der Außenwand des oberen Teils der Glockenform und der Vertikalen aufweist, wobei die Tangente an die Außenwand des Bodens des Hauptkörpers definiert und der Winkel kleiner oder gleich 20° und so angepasst ist, dass er das Einspritzen eines verwirbelten Gases erleichtert, und er ferner so konfiguriert ist, dass sich das Trockenpulver auf den in dem Zellträger kultivierten Zellen ablagert;
   wobei der Gasraum einen Pulverträger zum Einfüllen der Trockenpulver umfasst;
   wobei der genannte Pulverträger bevorzugt perforiert ist, um die Pulver zurückzuhalten und die Verteilung der Pulver im Luftstrom zu erleichtern.

2. Tragbare Vorrichtung nach dem vorangehenden Anspruch, wobei der Hauptkörper eine Glockenform mit einem Winkel zur Vertikalen umfasst, die durch die Tangente an die Außenwand des Bodens des Hauptkörpers definiert ist, wobei der Winkel im Bereich von 15° bis 19°, bevorzugt von 16° bis 18°, besonders bevorzugt von 17° bis 18° liegt.

3. Tragbare Vorrichtung nach einem der vorangehenden Ansprüche, wobei der Einlass für den Gasstrom einen Kanal zur Gaseinspritzung umfasst.

4. Tragbare Vorrichtung nach einem der vorangehenden Ansprüche, umfassend eine Dichtung im oberen Bereich des Gasraums, um das Entweichen der Trockenpulver zu verhindern.

5. Tragbare Vorrichtung nach einem der vorangehenden Ansprüche, wobei der Pulverträger auf derselben Höhe wie der Gaseinlass angeordnet ist und wobei der Pulverträger und/oder die Dichtung ferner aus einem Einwegmaterial gefertigt sind.

6. Tragbare Vorrichtung nach einem der vorangehenden Ansprüche, wobei der Druck des trockenen Fluids im Bereich von 200 MPa bis 415 MPa, bevorzugt von 210 MPa bis 310 MPa, besonders bevorzugt von 240 MPa bis 290 MPa liegt.

7. Tragbare Vorrichtung nach einem der vorangehenden Ansprüche, wobei der Hauptkörper eine Höhe von bis zu 20 cm aufweist, bevorzugt von 5 bis 20 cm und besonders bevorzugt von 10 bis 20 cm, wobei der Hauptkörper insbesondere an der Unterseite durch die Schwerkraft auf einem Zellträgermaterial fixiert wird.

8. Tragbare Vorrichtung nach einem der vorangehenden Ansprüche, wobei der Boden des Hauptkörpers einen Außendurchmesser von bis zu 4,15 cm, bevorzugt von 4,10 bis 3,85 cm, besonders bevorzugt von 3,90 bis 4,00 cm, und einen Innendurchmesser von 3,65 bis 3,80 cm, bevorzugt von 3,70 bis 3,75 cm, aufweist.

9. Tragbare Vorrichtung nach einem der vorangehenden Ansprüche, wobei der Einlass für den Gasstrom im Bereich von 1,5 bis 4,0 cm, bevorzugt im Bereich von 2,0 bis 3,5 cm liegt, und bevorzugt eine Länge von 2,5 bis 3,25 cm sowie eine Breite von 0,3 bis 0,8 cm, bevorzugt von 0,4 bis 0,7 cm, besonders bevorzugt von 0,5 bis 0,6 cm aufweist.

10. Tragbare Vorrichtung nach einem der vorangehenden Ansprüche, wobei das trockene Fluid Luft ist.

11. Tragbare Vorrichtung nach einem der vorangehenden Ansprüche, wobei der Zellträger eine Platte, eine Petrischale oder eine Mikrowaage mit Quarzkristall ist.

12. Tragbare Vorrichtung nach einem der vorangehenden Ansprüche, wobei die tragbare Vorrichtung aus einem Polymermaterial und/oder einem Verbundmaterial besteht, ausgewählt aus einer List bestehend aus: Acrylnitril-Butadien-Styrol, Polylactide, abwaschbarem Harz, Epoxidharz oder Kombinationen davon.

13. Tragbare Vorrichtung nach einem der vorangehenden Ansprüche, wobei die tragbare Vorrichtung durch additive Fertigung hergestellt wird, insbesondere durch Schmelzschichtung (FDM), Stereolithografie, 3D-Druck oder Formpressen.

14. Tragbare Vorrichtung nach einem der vorangehenden Ansprüche, ferner umfassend eine halbtransparente, flexible Folie, bestehend aus einer Mischung aus Wachsen und Polyolefinen, die sich im Verbindungsbereich zwischen dem Pulverträger und dem Hauptkörper befindet.

15. Verfahren zur *in vitro* Prüfung von Trockenpulvern, umfassend die Verwendung der tragbaren Vorrichtung gemäß einem der vorstehenden Ansprüche.

**Revendications**

1. Dispositif portatif adapté pour déterminer l'exposition de cellules à des poudres sèches comprenant un corps principal creux, une base creuse, un espace de tête et au moins une entrée d'écoulement de gaz,

dans lequel le corps principal a une forme de cloche ;
dans lequel le bas du corps principal est adapté pour s'ajuster à un support de cellule ;
dans lequel l'espace de tête a la forme d'un cône inversé ;
dans lequel l'espace de tête est lié au corps principal ;
dans lequel la forme de cloche du corps principal comprend un angle entre la paroi externe de la partie supérieure de la forme de cloche à la verticale défini par la tangente à la paroi externe du bas du corps principal inférieur ou égal à 20°, et qui est adaptée pour faciliter l'injection turbulente d'un gaz, et qui est configurée pour déposer la poudre sèche sur des cellules cultivées dans le support de cellule ;
dans lequel l'espace de tête comprend un support à poudre pour le chargement des poudres sèches ;
préférablement dans lequel ledit support de poudre est perforé pour retenir les poudres et faciliter la dispersion de poudre avec le flux d'air.

2. Dispositif portatif selon la revendication précédente, dans lequel le corps principal comprend une forme de cloche avec un angle à la verticale défini par la tangente à la paroi externe du bas du corps principal variant de 15° à 19°, plus préférablement de 16° à 18°, encore plus préférablement de 17° à 18°.

3. Dispositif portatif selon l'une quelconque des revendications précédentes, dans lequel l'entrée d'écoulement du gaz comprend un canal pour l'injection de gaz.

4. Dispositif portatif selon l'une quelconque des revendications précédentes, comprenant un joint sur le haut de l'espace

de tête pour prévenir l'échappement des poudres sèches.

**5.** Dispositif portatif selon l'une quelconque des revendications précédentes, dans lequel le support à poudre est placé à la même hauteur que l'entrée d'écoulement de gaz, et également dans lequel le support à poudre et/ou le joint sont faits à partir d'un matériel jetable.

**6.** Dispositif selon l'une quelconque des revendications précédentes, dans lequel la pression du fluide sec varie de 200 MPa à 415 Mpa, variant préférablement de 210 MPa à 310 MPa, variant plus préférablement de 240 MPa à 290 MPa.

**7.** Dispositif portatif selon l'une quelconque des revendications précédentes, dans lequel le corps principal comprend une hauteur pouvant aller jusqu'à 20 cm, préférablement variant de 5 à 20 cm et plus préférablement de 10 à 20 cm, en particulier dans lequel le corps principal est ancré au bas au-dessus d'un matériau de support de cellule par la gravité.

**8.** Dispositif portatif selon l'une quelconque des revendications précédentes, dans lequel le bas du corps principal comprend jusqu'à 4,15 cm, variant préférablement de 4,10 à 3,85 cm, plus préférablement variant de 3,90 à 4,00 cm de diamètre externe et variant de 3,65 à 3,80 cm, variant préférablement de 3,70 à 3,75 cm de diamètre interne.

**9.** Dispositif portatif selon l'une quelconque des revendications précédentes, dans lequel l'entrée pour l'écoulement de gaz varie de 1,5 à 4,0 cm, variant préférablement de 2,0 à 3,5 cm, variant plus préférablement de 2,5 à 3,25 cm de longueur et ayant une largeur variant de 0,3 à 0,8 cm, préférablement de 0,4 à 0,7 cm, encore plus préférablement de 0,5 à 0,6 cm.

**10.** Dispositif portatif selon l'une quelconque des revendications précédentes, dans lequel le fluide sec est de l'air.

**11.** Dispositif portatif selon l'une quelconque des revendications précédentes, dans lequel le support de cellule est un plat ou une boîte de Pétri ou une microbalance à cristal de quartz.

**12.** Dispositif portatif selon l'une quelconque des revendications précédentes, dans lequel le dispositif portatif est fait d'un matériau polymère et/ou d'un matériau composite choisi parmi une liste consistant en : acrylonitrile butadiène styrène, acide polylactique, résine lavable, époxy ou des combinaisons de ceux-ci.

**13.** Dispositif portatif selon l'une quelconque des revendications précédentes dans lequel le dispositif portatif est obtenu par fabrication additive, en particulier obtenu par modélisation des dépôts de fusion, stéreolithographie, impression 3D ou moulage.

**14.** Dispositif portatif selon l'une quelconque des revendications précédentes, comprenant également un film semi-transparent flexible composé d'un mélange de cires et polyoléfines dans la connexion entre le support à poudre et le corps principal.

**15.** Procédé de test *in vitro* de poudres sèches comprenant l'utilisation du dispositif portatif selon l'une quelconque des revendications précédentes.

Headspace
Air compressor
Main body
Dry powders
Plate with cells
Expected:
Device anchors here
Quartz-Crystal Microbalance

Fig. 1

A
B

Fig. 2

A
B

Fig. 3

100
80
60
40
20
0
Yield of aerosolization (%)
207
248
290
331
372
414
Air pressure (MPa)
XG MP
XG-Na MP
DS MP

Fig. 4

A549 3h
A549 24h
Cell viability (%)
140
130
120
110
100
90
80
70
60
50
40
30
20
10
0
D1 - Air
D1 - Air + MP LBG S 130
D2 - Air
D2 - Air + MP LBG S 130
Samples

Fig. 5

A B

Fig. 6

α

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011003022 A
- US 2010083737 A1


### Non-patent literature cited in the description


- A review of in vitro cigarette smoke exposure systems. *Experimental and Toxicologic Pathology*, 2013, vol. 65, 1183-1193
- **HE Y** ; **LIANG Y** ; **HAN R** ; **LU W-L** ; **MAK J C W** ; **ZHENG Y**. Rational particle design to overcome pulmonary barriers for obstructive lung diseases therapy. *J Control Release*, 2019, vol. 314, 48-61
- **ZIAEE A** ; **ALBADARIN A B** ; **PADRELA L** ; **FEMMER T** ; **O'REILLY E** ; **WALKER G**. Spray drying of pharmaceuticals and biopharmaceuticals: Critical parameters and experimental process optimization approaches. *Eur J Pharm Sci*, 2019, vol. 127, 300-18
- **MOLAVI F** ; **BARZEGAR-JALALI M** ; **HAMISHEHKAR H**. Polyester based polymeric nano and microparticles for pharmaceutical purposes: A review on formulation approaches. *J Control Release*, 2020, vol. 320, 265-282
- **FRÖHLICH E** ; **MERCURI A** ; **WU S** ; **SALAR-BEHZADI S**. Measurements of deposition, lung surface area and lung fluid for simulation of inhaled compounds. *Front Pharmacol*, 2016, vol. 7 (181)
- **DING Y** ; **WEINDL P** ; **LENZ A G** ; **MAYER P** ; **KREBS T** ; **SCHMID O**. Quartz crystal microbalances (QCM) are suitable for real-time dosimetry in nanotoxicological studies using VITROCELL®Cloud cell exposure systems. *Part Fibre Toxicol*, 2020, vol. 17, 44
- **KONJAC GLUCOMANNAN** ; **GUERREIRO, F** ; **PONTES, J.F** ; **ROSA DA COSTA, A.M** ; **GRENHA, A**. Spray-drying of konjac glucomannan to produce microparticles for an application as antitubercular drug carriers. *Powder Technology*, 2019, vol. 342, 246-252
- **CARRAGEENAN: RODRIGUES, S.** ; **CUNHA, L** ; **RICO, J.** ; **ROSA DA COSTA, A.M** ; **ALMEIDA, A.J** ; **FALEIRO, M.L** ; **BUTTINI, F** ; **GRENHA, A**. Carrageenan from red algae: An application in the development of inhalable tuberculosis therapy targeting the macrophages. *Drug Delivery and Translational Research*, 2020, vol. 10, 1675-1687
- **RODRIGUES, S** ; **ROSA DA COSTA, A.M** ; **FERNÁNDEZ-FLÓREZ, N** ; **TORRES, M.D.** ; **FALEIRO, M.L.** ; **BUTTINI, F** ; **GRENHA, A**. Inhalable spray-dried chondroitin sulphate microparticles: Effect of different solvents on particle properties and drug activity. *Polymers*, 2020, vol. 12
- **ALVES, A.D** ; **CAVACO, J.S.** ; **GUERREIRO, F** ; **LOURENCO, J.P** ; **ROSA DA COSTA, A.M.** ; **GRENHA, A**. Inhalable antitubercular therapy mediated by locust bean gum microparticles. *Molecules*, 2016, vol. 21 (702)